(19) Europäisches Patentamt
European Patent Office
Office européen des brevets

(11) Veröffentlichungsnummer: **0 685 480 A1**

# (12) EUROPÄISCHE PATENTANMELDUNG

(21) Anmeldenummer: **95106233.0**

(22) Anmeldetag: **26.04.95**

(51) Int. Cl.[6]: **C07D 498/04**, C09B 19/02, C09B 62/04, D06P 3/66

(30) Priorität: **04.05.94 DE 4415692**

(43) Veröffentlichungstag der Anmeldung: **06.12.95 Patentblatt 95/49**

(84) Benannte Vertragsstaaten: **AT BE CH DE ES FR GB IT LI NL PT**

(71) Anmelder: **HOECHST AKTIENGESELLSCHAFT**
**Brüningstrasse 50**
**D-65929 Frankfurt am Main (DE)**

(72) Erfinder: **Russ, Werner Hubert, Dr.**
**Wingertstrasse 8a**
**D-65439 Flörsheim (DE)**
Erfinder: **Schumacher, Christian, Dr.**
**Johannesallee 41**
**D-65929 Frankfurt am Main (DE)**

## (54) Wasserlösliche Triphendioxazinverbindungen, Verfahren zu deren Herstellung und ihre Verwendung als Farbstoffe.

(57) Es werden Triphendioxazinverbindungen entsprechend der allgemeinen Formel (1)

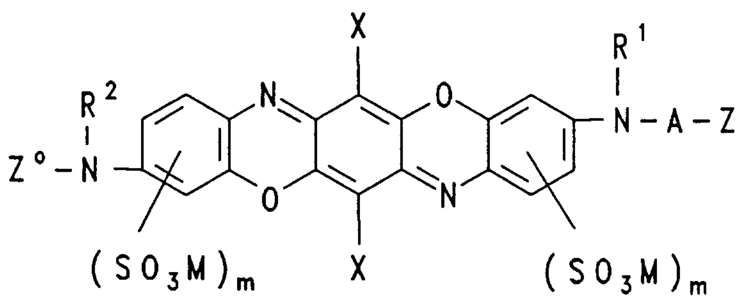

(1)

beschrieben, in welcher M Wasserstoff oder ein Alkalimetall ist, m die Zahl 1 oder 2 ist, X Wasserstoff, gegebenenfalls substituiertes Alkyl, Aryl oder Aryloxy oder Cyano, Alkoxycarbonyl, Aryloxycarbonyl, Aminocarbonyl, alkyliertes Aminocarbonyl, gegebenenfalls substituiertes Alkyl- oder Arylcarbonyl oder insbesondere Halogen ist, $R^1$ und $R^2$ jedes Wasserstoff oder gegebenenfalls substituiertes Alkyl oder Aryl ist, Z ein Rest der allgemeinen Formel (2)

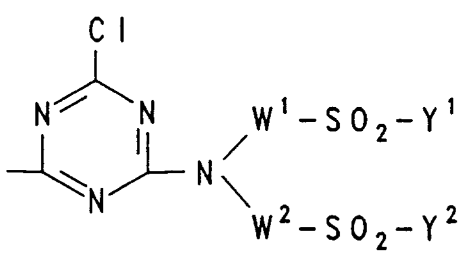

(2)

ist, in welcher $Y^1$ und $Y^2$ jedes Vinyl, $\beta$-Hydroxyethyl oder ein in $\beta$-Stellung einen alkalisch eliminierbaren

EP 0 685 480 A1

Substituenten enthaltendes Ethyl ist, wobei $Y^1$ und $Y^2$ nicht gleichzeitig $\beta$-Hydroxyethyl bedeuten, $W^1$ und $W^2$ beide Alkylen von 2 bis 6 C-Atomen bedeuten, A eine kovalente Bindung oder eine Gruppe der Formel $-B^1-N(R^3)-$ ist, in welcher $R^3$ eine der Bedeutungen von $R^1$ hat und $B^1$ ein aliphatisches oder aromatisches Brückenglied ist, wobei die Gruppe $-N(R^1)-B^1-N(R^3)-$ auch insgesamt einen bivalenten gesättigten heterocyclischen Rest darstellen kann, und $Z^0$ Wasserstoff oder eine Gruppe der allgemeinen Formel (4)

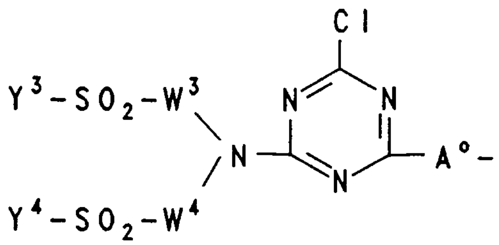

(4)

ist, in welcher $Y^3$, $Y^4$, $W^3$ und $W^4$ eine der für $Y^1$ bzw. $W^1$ genannten Bedeutungen besitzt und $A^0$ eine kovalente Bindung oder ein Rest der Formel $-N(R^4)-B^2-$ mit der der obigen Formel $-B^1-(NR^3)-$ entsprechenden Bedeutung ist.

Die Triphendioxazinverbindungen der Formel (1) dienen als faserreaktive Farbstoffe zum Färben von hydroxy-, amino- und/oder carbonamidgruppenhaltigem Material, insbesondere Fasermaterial, wie Cellulosefasern, hiervon insbesondere Baumwolle, Wolle oder andere Tierhaare und Nylon.

Die Erfindung liegt auf dem technischen Gebiet der faserreaktiven Farbstoffe.

Aus der britischen Patentschrift 1 576 237 und den europäischen Patentanmeldungs-Veröffentlichungen Nrs. 0 070 807 und 0 074 928 sind Farbstoffe, jedoch keine Triphendioxazinfarbstoffe, bekannt, die einen {Bis-[β-(β'-chlorethylsulfonyl)-ethyl]-amino}- oder {Bis-[β-(β'-sulfatoethylsulfonyl)-ethyl]-amino}-chlor-s-triazinyl- oder -fluor-s-triazinyl-Rest als faserreaktive Gruppe enthalten. Sofern in diesen Druckschriften Triphendioxazinfarbstoffe beschrieben werden, enthalten sie als faserreaktive Gruppierung einen [β-(β'-Sulfatoethylsulfonyl)-ethylamino]-, [β-(β'-Chlorethylsulfonyl)-ethylamino]-, [γ-(β'-Sulfatoethylsulfonyl)-propylamino]- oder [γ-(β'-Chlorethylsulfonyl)-propylamino]-chlor-s-triazinyl- oder -fluor-s-triazinyl-Rest oder den 2-{Bis-[β-(β'-sulfatoethylsulfonyl)-ethyl]-amino}-4-fluor-1,3,5-triazin-6-yl-amino-Rest. Triphendioxazinfarbstoffe dieser Art besitzen jedoch gewisse anwendungstechnische Nachteile, teils wegen ihrer schlechten Wasserlöslichkeit oder einer zu hohen Fasersubstantivität.

Mit der vorliegenden Erfindung wurden nunmehr neue Triphendioxazinverbindungen mit guten faserreaktiven Farbstoffeigenschaften gefunden, die der allgemeinen Formel (1)

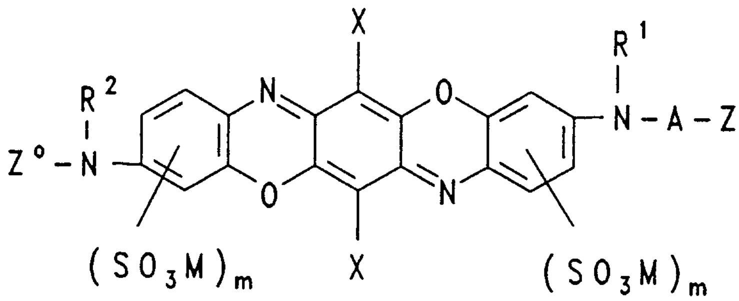

(1)

entsprechen. In dieser Formel bedeuten:

M ist Wasserstoff oder ein Alkalimetall, wie Natrium, Kalium oder Lithium;

m ist die Zahl 1 oder 2, bevorzugt 1, wobei eine Gruppe $-SO_3M$ bevorzugt in ortho-Stellung zur Oxigruppe gebunden ist;

X ist Wasserstoff, Halogen, wie Chlor oder Brom, gegebenenfalls substituiertes Alkyl von 1 bis 4 C-Atomen, wie Methyl oder Ethyl, gegebenenfalls substituiertes Aryl, wie Phenyl oder Naphthyl, gegebenenfalls substituiertes Aryloxy, wie Phenoxy oder Naphthoxy, Cyano, Alkoxycarbonyl von 2 bis 5 C-Atomen, wie Methoxycarbonyl und Ethoxycarbonyl, gegebenenfalls substituiertes Aryloxycarbonyl, wie Phenoxycarbonyl oder Naphthoxycarbonyl, Aminocarbonyl, N-Alkyl-aminocarbonyl mit einem Alkylrest von 1 bis 4 C-Atomen, N,N-Dialkyl-aminocarbonyl mit Alkylresten von jeweils 1 bis 4 C-Atomen, gegebenenfalls substituiertes Alkylcarbonyl von 2 bis 5 C-Atomen, wie Acetyl oder Propionyl, oder gegebenenfalls substituiertes Arylcarbonyl, und ist bevorzugt Chlor, Methyl, Ethyl, Methoxy oder Phenyl und insbesondere bevorzugt Chlor;

$R^1$ ist Wasserstoff oder gegebenenfalls substituiertes Alkyl von 1 bis 6 C-Atomen, bevorzugt von 1 bis 4 C-Atomen, oder ist gegebenenfalls substituiertes Aryl und ist bevorzugt Wasserstoff;

$R^2$ hat eine der für $R^1$ angegebene Bedeutungen;

Z ist ein Rest der allgemeinen Formel (2)

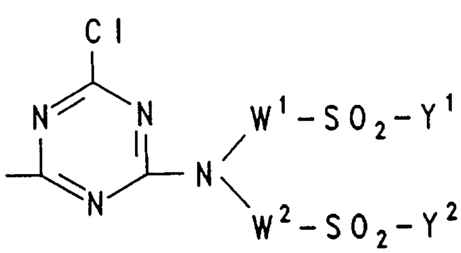

(2)

in welcher

$Y^1$ Vinyl ist oder Ethyl ist, das in β-Stellung einen Substituenten enthält, der mittels Alkali unter Bildung der Vinylgruppe eliminierbar ist, oder β-Hydroxyethyl ist,

$Y^2$ eine der Bedeutungen von $Y^1$ hat und bevorzugt die gleiche Bedeutung wie $Y^1$ besitzt und mit der Maßgabe, daß $Y^1$ und $Y^2$ nicht gleichzeitig β-Hydroxyethyl bedeuten,

$W^1$ Alkylen von 2 bis 6 C-Atomen und bevorzugt von 2 oder 3 C-Atomen bedeutet, wie insbesondere 1,2-Ethylen oder 1,3-Propylen, und

$W^2$ eine der für $W^1$ angegebenen Bedeutungen hat und bevorzugt die gleiche Bedeutung wie $W^1$ besitzt;

A ist eine kovalente Bindung oder eine Gruppe der allgemeinen Formel (3a)

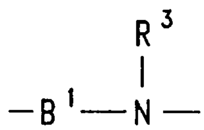

(3a)

in welcher

$R^3$ eine der für $R^1$ angegebenen Bedeutungen besitzt und

$B^1$ Alkylen von 2 bis 6 C-Atomen, bevorzugt von 2 oder 3 C-Atomen, wie 1,2-Ethylen und 1,3-Propylen, ist oder Alkylen von 3 bis 10 C-Atomen, bevorzugt von 4 bis 8 C-Atomen, ist, das durch 1 oder 2 Heterogruppen, wie Gruppen der Formeln -O- , -NH- , -S- , $-SO_2-$, -CO-, -NH-CO-, $-NH-SO_2-$, $-SO_2-NH-$, -CO-NH-, -NH-CO-NH-, -NH-CO-O-, -CO-O-, -O-OC-NH-, -O-OC- oder -N(R)- mit R gleich Alkyl von 1 bis 4 C-Atomen, wie Ethyl und Methyl, unterbrochen ist, oder Cycloalkylen von 5 bis 8 C-Atomen, wie Cyclohexylen, ist, oder ein Rest der Formel phen, naphth , alk-phen, phen-alk, naphth-alk, alk-naphth, phen-alk-phen, alk-phen-alk, phen-D-phen, alk-D-phen, phen-D-alk, cy-alk, alk-cy, cy-alk-cy oder alk-cy-alk, in welchen phen Phenylen bedeutet, das durch 1 oder 2 Substituenten aus der Gruppe Methyl, Ethyl, Methoxy, Sulfo und Carboxy substituiert sein kann, naphth Naphthylen bedeutet, das durch 1 oder 2 Sulfogruppen substituiert sein kann, alk Alkylen von 1 bis 6 C-Atomen, bevorzugt von 2 bis 4 C-Atomen, bedeutet, das durch 1 oder 2 Substituenten aus der Gruppe Hydroxy, Acetyloxy, Sulfo, Carboxy oder Sulfato substituiert und/oder durch eine der obengenannten Heterogruppen unterbrochen sein kann, cy Cycloalkylen von 5 bis 8 C-Atomen, wie Cyclohexylen, ist und D eine der obengenannten Heterogruppen bedeutet, oder

die Gruppe $-N(R^1)-B^1-N(R^3)-$ stellt insgesamt einen bivalenten gesättigten heterocyclischen Rest dar, wie den 1,4-Piperazinylen-Rest;

$Z^0$ ist Wasserstoff oder ist eine Gruppe der allgemeinen Formel (4)

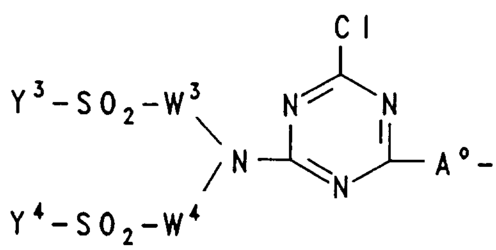

(4)

in welcher

$Y^3$ und $Y^4$ eine der oben für $Y^1$ genannten Bedeutungen haben und $Y^3$ und $Y^4$ bevorzugt zueinander die gleiche Bedeutung besitzen,

$W^3$ und $W^4$ eine der oben für $W^1$ genannten Bedeutungen haben und $W^3$ und $W^4$ bevorzugt zueinander die gleiche Bedeutung besitzen,

$A^0$ eine kovalente Bindung oder ein Rest der allgemeinen Formel (3b)

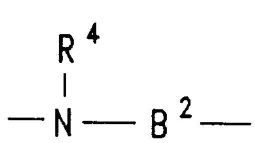

(3b)

ist, in welcher

$R^4$ eine der oben für $R^1$ angegebenen Bedeutungen hat und

$B^2$ eine der oben für $B^1$ genannten Bedeutungen besitzt, oder

die Gruppe $-N(R^4)-B^2-N(R^2)-$ stellt einen bivalenten gesättigten heterocyclischen Rest, wie den 1,4-Piperazinylen-Rest, dar.

In den obengenannten sowie auch nachfolgenden allgemeinen Formeln können die einzelnen Formelglieder, sowohl verschiedener als auch gleicher Bezeichnung, innerhalb einer allgemeinen Formel im Rahmen ihrer Bedeutung zueinander gleiche oder voneinander verschiedene Bedeutungen besitzen.

$\beta$-ständige Substituenten in den obengenannten Ethylgruppen Y sind beispielsweise Sulfato, Thiosulfato, Phosphato, Alkanoyloxy von 2 bis 5 C-Atomen, wie Acetyloxy, Benzoyloxy, Sulfobenzoyloxy, p-Toluolsulfonyloxy oder Halogen, wie Chlor oder Brom, hiervon bevorzugt Sulfato und insbesondere Chlor. Die Gruppen Y stehen weiterhin bevorzugt für Vinyl.

Die Gruppen "Sulfo", "Carboxy", "Phosphato", "Thiosulfato" und "Sulfato" schließen sowohl deren Säureform als auch deren Salzform ein. Demgemäß bedeuten Sulfogruppen Gruppen entsprechend der allgemeinen Formel $-SO_3M$ , Carboxygruppen Gruppen entsprechend der allgemeinen Formel -COOM , Phosphatogruppen Gruppen entsprechend der allgemeinen Formel $-OPO_3M_2$ , Thiosulfatogruppen Gruppen entsprechend der allgemeinen Formel $-S-SO_3M$ und Sulfatogruppen Gruppen entsprechend der allgemeinen Formel $-OSO_3M$ , in welchen M die obengenannte Bedeutung hat.

Substituenten in den für X, $R^1$, $R^2$, $R^3$ und $R^4$ stehenden substituierten Alkylgruppen sind beispielsweise Methoxy, Ethoxy, Hydroxy, Alkanoyloxy von 2 bis 5 C-Atomen, wie Acetyloxy, Sulfo, Carboxy, Sulfato und Chlor. Arylreste sind beispielsweise Phenylreste oder Naphthylreste. Substituierte Arylreste sind beispielsweise solche, die durch 1, 2 oder 3 Substituenten aus der Gruppe Sulfo, Carboxy, Methoxy, Ethoxy, Methyl, Ethyl, Chlor und Brom substituiert sind. Substituierte Naphthylreste sind insbesondere solche, die durch 1 bis 3 Sulfogruppen und/oder eine Carboxygruppe substituiert sind.

Reste $W^1$, $W^2$, $W^3$ und $W^4$ sind beispielsweise 1,2-Ethylen, 1,3-Propylen, 1,4-Butylen, 1,5-Pentylen, 1,6-Hexylen, 2-Methyl-1,3-propylen und Reste der Formeln $-CH_2-CH(CH_3)-$, $-CH(CH_3)-CH_2-$ und $-(CH_2)_2-O-(CH_2)_2-$.

Reste $B^1$ sind beispielsweise 1,2-Ethylen, 1,3-Propylen, 1,4-Butylen, 1,5-Pentylen, 1,6-Hexylen, 2-Methyl-1,3-propylen, 1,4-Cyclohexylen, 1,4-Phenylen, 1,3-Phenylen, 2,5-Disulfo-1,4-phenylen, 2,5-Disulfo-1,3-phenylen, 3-Sulfo-1,4-phenylen, 2-Sulfo-1,4-phenylen, 5-Sulfo-1,3-phenylen und Reste der Formeln $-CH_2-CH(CH_3)-$, $-CH(CH_3)-CH_2-$, $-(CH_2)_2-NH-(CH_2)_2-$, $-(CH_2)_3-NH-(CH_2)_3-$, $-(CH_2)_2-NH-(CH_2)_3-$, $-(CH_2)_3-NH-(CH_2)_2-$, $-(CH_2)_2-O-(CH_2)_2-$, $-(CH_2)_2-O-(CH_2)_3-$, $-(CH_2)_3-O-(CH_2)_3-$, $-(CH_2)_3-O-(CH_2)_2-$, $-(CH_2)_2-NH-CO-(CH_2)_2-$, $-(CH_2)_2-NH-SO_2-(CH_2)_2-$, $-(CH_2)_2-CO-NH-(CH_2)_2-$, $-(CH_2)_2-SO_2-NH-(CH_2)_2-$, $-(CH_2)_2-COO-(CH_2)_2-$, $-(CH_2)_2-OOC-(CH_2)_2-$, $-(CH_2)_2-NH-COO-(CH_2)_2-$ oder $-(CH_2)_2-NH-CO-NH-(CH_2)_2-$ oder der Formel

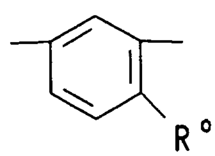

in welcher $R^0$ Methoxy oder Methyl ist. Bevorzugt ist $B^1$ Ethylen oder Propylen oder durch 1 oder 2 Sulfo substituiertes Phenylen.

Reste $B^2$ sind beispielsweise 1,2-Ethylen, 1,3-Propylen, 1,4-Butylen, 1,5-Pentylen, 1,6-Hexylen, 2-Methyl-1,3-propylen, 1,4-Cyclohexylen, 1,4-Phenylen, 1,3-Phenylen, 2,5-Disulfo-1,4-phenylen, 2,5-Disulfo-1,3-phenylen, 3-Sulfo-1,4-phenylen, 2-Sulfo-1,4-phenylen, 5-Sulfo-1,3-phenylen, und Reste der Formeln $-CH_2-CH(CH_3)-$, $-CH(CH_3)-CH_2-$, $-(CH_2)_2-NH-(CH_2)_2-$, $-(CH_2)_3-NH-(CH_2)_3-$, $-(CH_2)_2-NH-(CH_2)_3-$, $-(CH_2)_3-NH-(CH_2)_2-$, $-(CH_2)_2-O-(CH_2)_2-$, $-(CH_2)_2-O-(CH_2)_3-$, $-(CH_2)_3-O-(CH_2)_3-$, $-(CH_2)_3-O-(CH_2)_2-$, $-(CH_2)_2-NH-CO-(CH_2)_2-$, $-(CH_2)_2-NH-SO_2-(CH_2)_2-$, $-(CH_2)_2-CO-NH-(CH_2)_2-$, $-(CH_2)_2-SO_2-NH-(CH_2)_2-$, $-(CH_2)_2-COO-(CH_2)_2-$, $-(CH_2)_2-OOC-(CH_2)_2-$, $-(CH_2)_2-NH-COO-(CH_2)_2-$ oder $-(CH_2)_2-NH-CO-NH-(CH_2)_2-$ oder der Formel

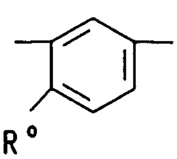

mit $R^0$ gleich Methoxy oder Methyl. Bevorzugt ist $B^2$ Ethylen oder Propylen oder durch 1 oder 2 Sulfo substituiertes Phenylen.

Reste X sind beispielsweise Chlor, Brom, Fluor, Jod, Methyl, Ethyl, n-Propyl, Isopropyl, Methoxy, Ethoxy, Phenyl, 4-Methyl-phenyl, 2-Methyl-phenyl, 2-Chlor-phenyl, 4-Chlor-phenyl, 4-Sulfo-phenyl, 3-Sulfo-phenyl, Phenoxy, 4-Methyl-phenoxy, 4-Sulfo-phenoxy, 3-Sulfo-phenoxy, Cyclohexyl, Cyclopentyl, Benzyl, Cyano, Ethoxycarbonyl, Methoxycarbonyl, Phenoxycarbonyl, Acetyl, Propionyl, Phenylcarbonyl, Amino, Methylamino, Dimethylamino, Ethylamino und Diethylamino.

Von den Triphendioxazinverbindungen der allgemeinen Formel (1) sind solche besonders bevorzugt, in welchen A eine kovalente Bindung ist, X für Chlor steht und $Z^0$ für Wasserstoff steht und $R^1$ und $R^2$ jeweils Wasserstoff bedeuten.

Von den Triphendioxazinverbindungen der allgemeinen Formel (1) sind weiterhin solche bevorzugt, in welchen A einen Rest der allgemeinen Formel (3a) ist, X für Chlor steht und $Z^0$ für Wasserstoff oder einen Rest der allgemeinen Formel (4) mit $A^0$ gleich einem Rest der allgemeinen Formel (3b) steht. Von diesen Verbindungen sind wiederum bevorzugt solche, in welchen $R^1$, $R^2$, $R^3$ und $R^4$ jeweils Wasserstoff bedeuten und $B^1$ oder $B^2$ oder beide eine der bevorzugten Bedeutungen haben.

Weiterhin sind solche Triphendioxazinverbindungen der allgemeinen Formel (1) hervorzuheben, in welchen deren Formelrest (5)

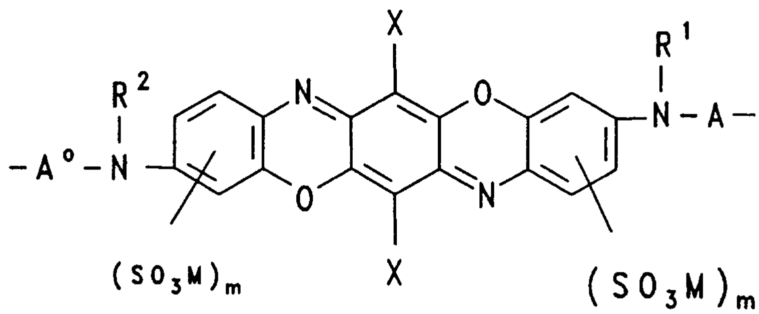

(5)

ein Rest der Formel (5A), (5B), (5C), (5D), (5E), (5F) oder (5G)

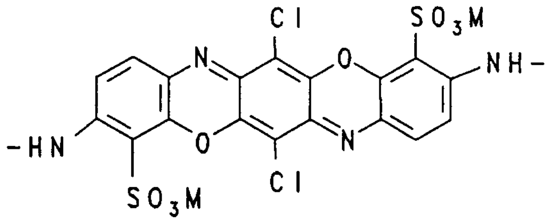

(5A)

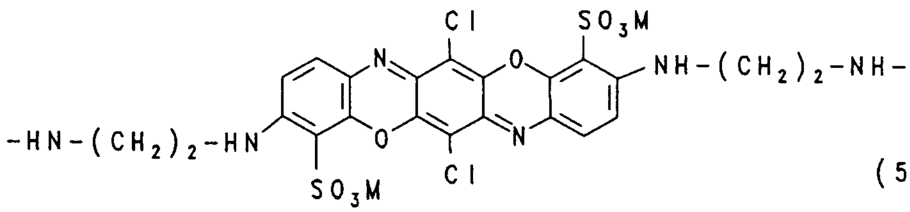

(5B)

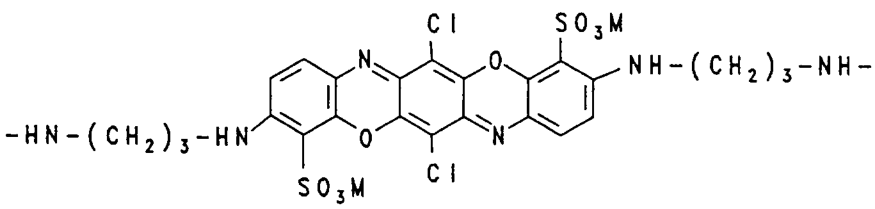

(5C)

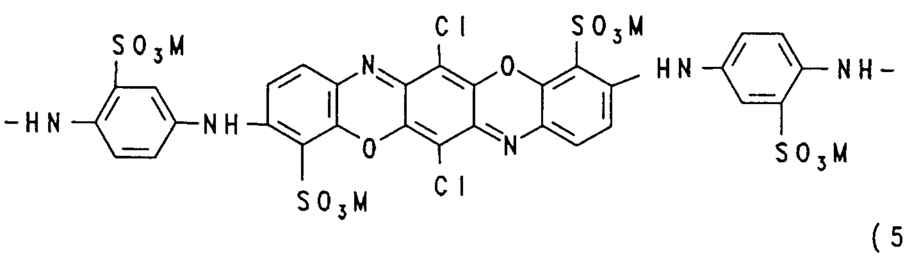

(5D)

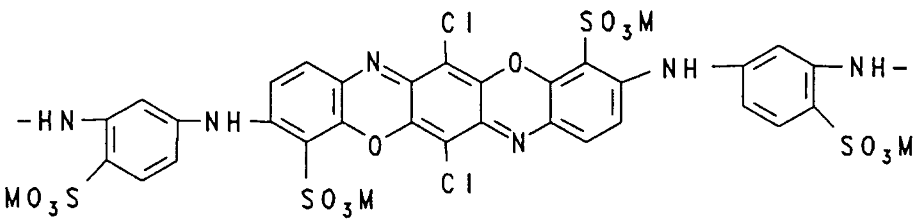

(5E)

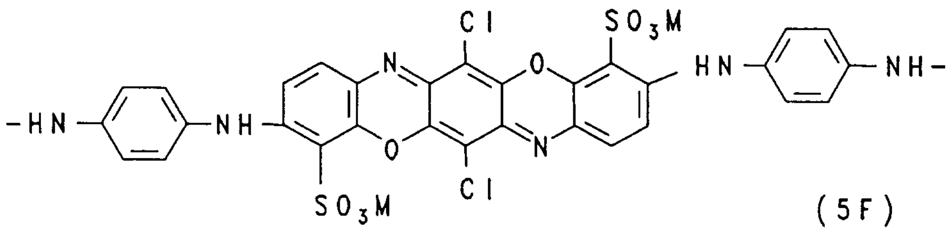

(5F)

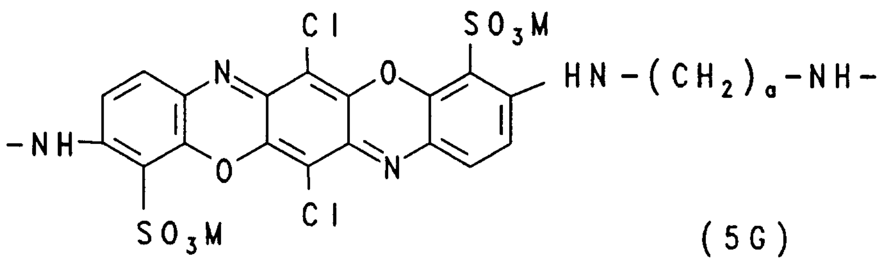

(5G)

ist, in welchen M die obengenannte Bedeutung besitzt und a für die Zahl 2 oder 3 steht.

Reste der allgemeinen Formeln (6a) und (6b)

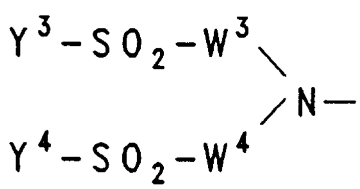

(6a)

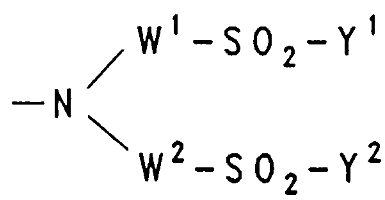

(6b)

in den Triphendioxazinverbindungen der allgemeinen Formel (1) sind beispielsweise Bis-N,N-[β-(β'-chlorethylsulfonyl)-ethyl]-amino, Bis-N,N-[β-vinylsulfonyl-ethyl]-amino, Bis-N,N-[β-(β'-sulfatoethylsulfonyl)-ethyl]-amino, Bis-N,N-[γ-(β'-chlorethylsulfonyl)-propyl]-amino, Bis-N,N-[γ-vinylsulfonyl-propyl]-amino, Bis-N,N-[γ-(β'-sulfatoethylsulfonyl)-propyl]-amino, Bis-N,N-[β-(β'-chlorethylsulfonyl)-isopropyl]-amino, Bis-N,N-[β-vinylsulfonyl-isopropyl]-amino, Bis-N,N-[β-(β'-sulfatoethylsulfonyl)-isopropyl]-amino, Bis-N,N-[δ-(β'-chlorethylsulfonyl)-butyl]-amino, Bis-N,N-[γ-(β'-chlorethylsulfonyl)-isobutyl]-amino und Bis-N,N-[β-(β'-chlorethylsulfonyl)-isobutyl]-amino.

Insbesondere sind erfindungsgemäße Triphendioxazinverbindungen hervorzuheben, die den allgemeinen Formeln (1A), (1B), (1C), (1D) und (1E)

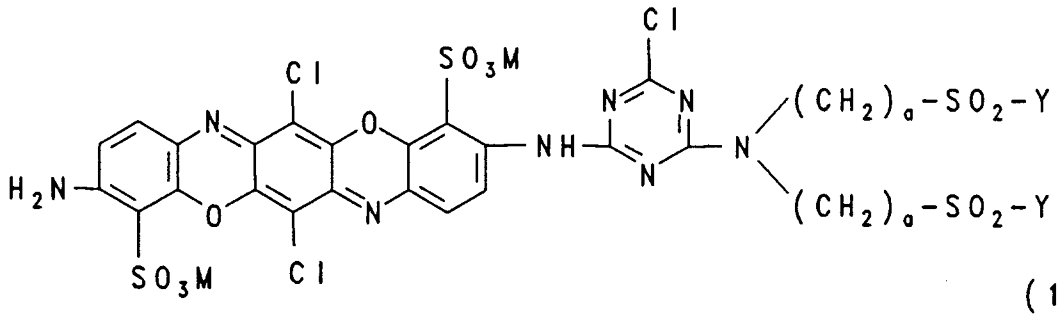

(1A)

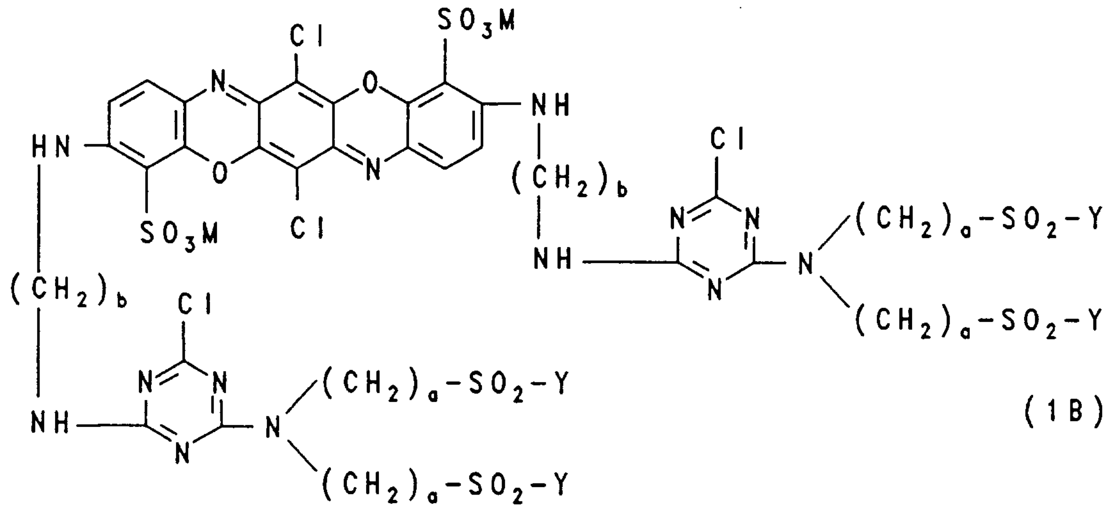

(1B)

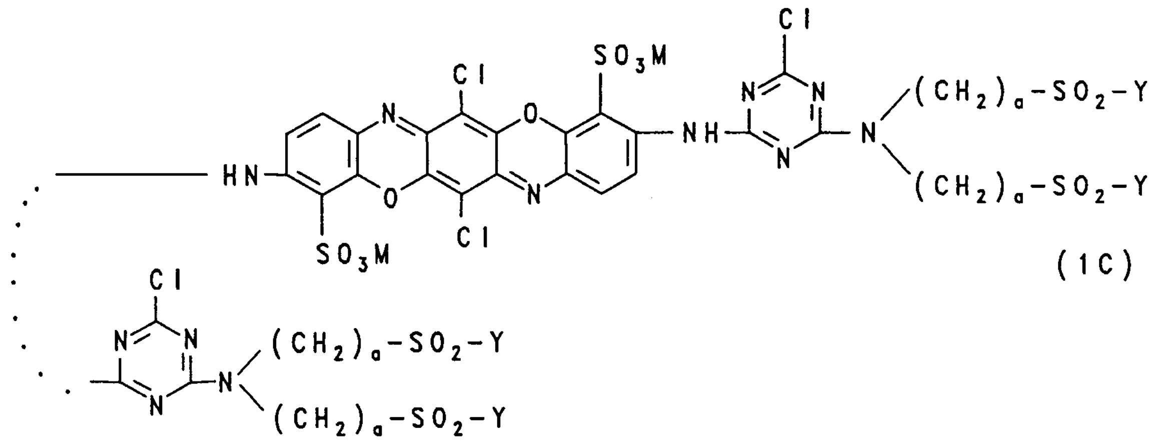

(1C)

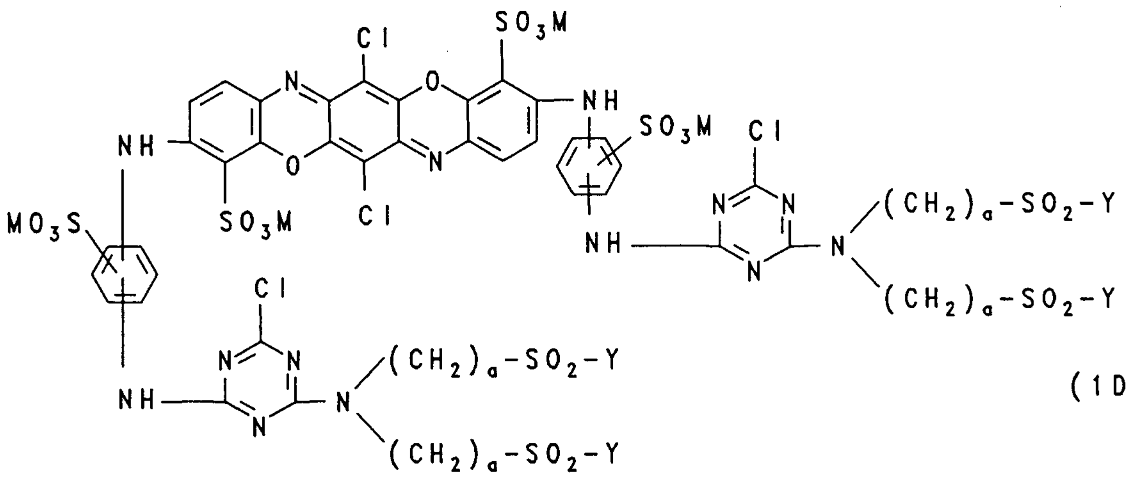

(1D)

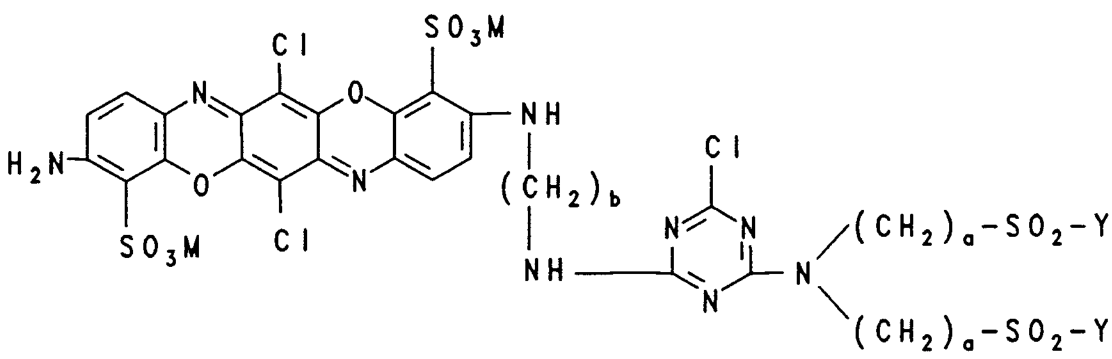

(1E)

entsprechen, in welchen M die obengenannte Bedeutung besitzt, a für die Zahl 2 oder 3 steht, b die Zahl 2 oder 3 bedeutet und Y $\beta$-Chlorethyl oder Vinyl ist, wobei die zwei bzw. vier Gruppen Y jeweils zueinander gleich oder voneinander verschieden sein können.

Die vorliegende Erfindung betrifft weiterhin ein Verfahren zur Herstellung der erfindungsgemäßen Triphendioxazinverbindungen der allgemeinen Formel (1), das dadurch gekennzeichnet ist, daß man

Cyanurchlorid (2,4,6-Trichlor-1,3,5-triazin) in beliebiger Reihenfolge mit einer aminogruppenhaltigen Triphendioxazinverbindung der allgemeinen Formel (7)

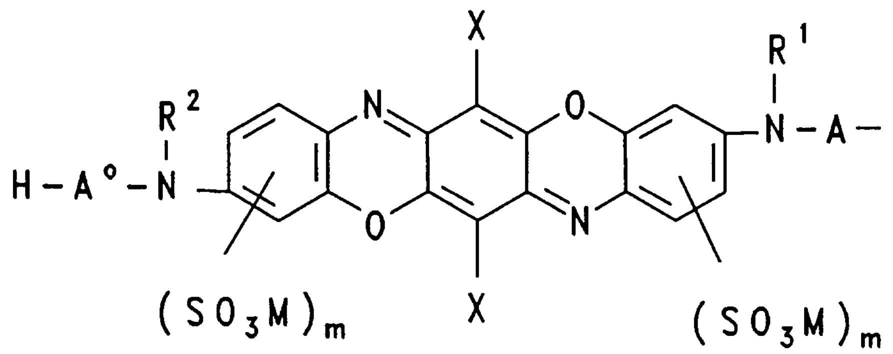

(7)

mit $A^0$, A, X, M, m, $R^1$ und $R^2$ der oben angegebenen Bedeutung und mit einem Amin der allgemeinen Formel (8a) und/oder (8b)

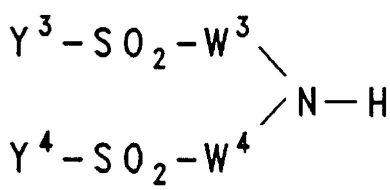

(8a)

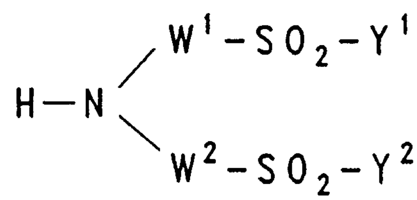

(8b)

mit $W^1$, $W^2$, $W^3$, $W^4$, $Y^1$, $Y^2$, $Y^3$ und $Y^4$ der oben angegebenen Bedeutung oder mit einem Salz des Amins einer in der Technik üblichen anorganischen oder organischen Säure, wie der Salzsäure, Schwefelsäure, Phosphorsäure oder Essigsäure, in der äquivalenten Menge bei einer Temperatur zwischen 0 °C und 100 °C und bei einem pH-Wert zwischen 2 und 10, bevorzugt zwischen 4 und 9, umsetzt.

Erfindungsgemäße Triphendioxazinverbindungen der allgemeinen Formel (1), in welcher A eine kovalente Bindung ist und $Z^0$ eine Gruppe der allgemeinen Formel (4) mit $A^0$ gleich einer kovalenten Bindung ist, werden bevorzugt erfindungsgemäß in der Weise hergestellt, daß man eine Triphendioxazinverbindung der allgemeinen Formel (7), in welcher A und $A^0$ beide eine kovalente Bindung bedeuten, mit einer Verbindung der allgemeinen Formel (9)

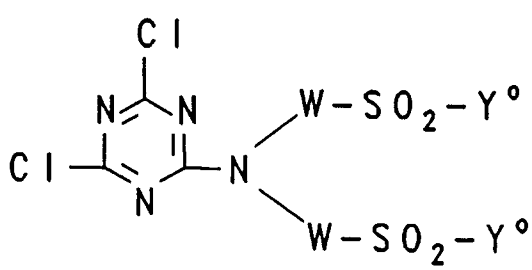

(9)

in welcher jedes W, zueinander gleich oder voneinander verschieden, und jedes $Y^0$, zueinander gleich oder voneinander verschieden, eine der Bedeutungen von $W^1$, $W^2$, $W^3$ und $W^4$ bzw. $Y^1$, $Y^2$, $Y^3$ und $Y^4$ haben, unter Einsatz der 4- bis 6-fach molaren Menge an der Verbindung (9) bei einer Temperatur zwischen 60 und 95 °C, bevorzugt zwischen 70 und 90 °C, und bei einem pH-Wert von 4 bis 5 umsetzt.

Erfindungsgemäße Triphendioxazinfarbstoffe der allgemeinen Formel (1), in welchen $Z^0$ Wasserstoff ist und A für eine kovalente Bindung steht, werden erfindungsgemäß bevorzugt in der Weise hergestellt, daß man eine Verbindung der allgemeinen Formel (7) mit A und $A^0$ gleich einer kovalenten Bindung mit einer Verbindung der allgemeinen Formel (9) der oben angegebenen Bedeutung bei einer Temperatur zwischen

60 und 95 ° C, bevorzugt zwischen 70 und 90 ° C, und bei einem pH-Wert zwischen 3 und 6, bevorzugt zwischen 4 und 5, unter Einsatz der 2- bis 3-fach molaren Menge der Verbindung (9) umsetzt.

Erfindungsgemäße Triphendioxazinverbindungen der allgemeinen Formel (1), in welcher A einen Rest der allgemeinen Formel (3a) und $Z^0$ ein Rest der allgemeinen Formel (4) mit $A^0$ gleich einem Rest der allgemeinen Formel (3b) ist, werden erfindungsgemäß bevorzugt in der Weise hergestellt, daß man eine Triphendioxazinverbindung der allgemeinen Formel (7), in welcher A und $A^0$ die eben gerade angegebene Bedeutungen haben, zunächst mit Cyanurchlorid bei einer Temperatur zwischen -5 ° C und 20 ° C, bevorzugt zwischen 0 und 10 ° C, und bei einem pH-Wert zwischen 8 und 10, bevorzugt zwischen 8,5 und 9, umsetzt und die so erhaltene Bis-(dichlor-triazinylamino)-triphendioxazin-Verbindung mit einer Aminoverbindung der allgemeinen Formel (8a) und/oder (8b) bei einer Temperatur zwischen 60 und 90 ° C, bevorzugt zwischen 70 und 80 ° C, und bei einem pH-Wert zwischen 5 und 8, bevorzugt zwischen 6 und 7, umsetzt, oder daß man eine Triphendioxazinverbindung der allgemeinen Formel (7) mit A und $A^0$ der eben angegebenen Bedeutung mit einer Verbindung der allgemeinen Formel (9) bei einer Temperatur zwischen 60 und 95 ° C, bevorzugt zwischen 70 und 90 ° C, und einem pH-Wert zwischen 3 und 6, bevorzugt zwischen 4 und 5, umsetzt.

Die Umsetzungen der Triphendioxazinverbindungen der allgemeinen Formel (7) mit Cyanurchlorid oder mit einer Verbindung der allgemeinen Formel (9) oder die Umsetzung der Bis-(dichlor-triazinylamino)-triphendioxazin-Verbindung mit einem Amin (8a) und/oder (8b) können sowohl in wäßrigem als auch in wäßrigorganischem Medium in Suspension oder Lösung erfolgen. Führt man die Umsetzungen in einem wäßrig-organischen Medium durch, so kann das organische Medium, das beispielsweise Aceton, Dimethylformamid, Dimethylacetamid, Dimethylsulfoxid oder N-Methyl-pyrrolidon ist, in einem Mischungsverhältnis von bis zu 40 Vol.-% vorliegen. Vorteilhaft wird bei der Kondensation frei werdende Salzsäure laufend durch Zugabe wäßriger Alkali-oder Erdalkalihydroxide, -carbonate oder -bicarbonate neutralisiert.

Die Ausgangsverbindungen sind beispielsweise aus den anfangs genannten Druckschriften sowie aus den U.S.-Patentschriften 3 883 523 und 4 568 742 und der Europäischen Patentschrift 0 101 665 bekannt oder können analog den dort gemachten Angaben hergestellt werden.

Die Abscheidung der erfindungsgemäß hergestellten Verbindungen der allgemeinen Formel (1) aus den Syntheseansätzen erfolgt nach allgemein bekannten Methoden entweder durch Ausfällen aus dem Reaktionsmedium mittels Elektrolyten, wie beispielsweise Natriumchlorid oder Kaliumchlorid, oder durch Eindampfen der Reaktionslösung, beispielsweise durch Sprühtrocknung, wobei dieser Reaktionslösung eine Puffersubstanz zugeführt werden kann.

Die Verbindungen der allgemeinen Formel (1) - im nachfolgenden als "Farbstoffe (1)" bezeichnet - haben faserreaktive Farbstoffeigenschaften und können zum Färben (einschließlich Bedrucken) von hydroxy-, amino- und/oder carbonamidgruppenhaltigen Materialien, insbesondere Fasermaterialien, verwendet werden. Gegenstand der vorliegenden Erfindung ist deshalb auch die Verwendung der Farbstoffe (1) zum Färben solcher Materialien bzw. Verfahren zum Färben solcher Materialien, bei welchem der Farbstoff (1) in gelöster Form auf das Material oder in das Material eingebracht und mittels Wärme oder unter Einwirkung eines alkalisch wirkenden Agenz oder mit Hilfe beider Maßnahmen fixiert wird.

Hydroxygruppenhaltige Materialien sind solche natürlichen oder synthetischen Ursprungs, wie beispielsweise Cellulosefasermaterialien, wie natürliche Cellulosefasern, oder deren Regeneratprodukte und Polyvinylalkohole. Cellulosefasermaterialien sind vorzugsweise Baumwolle, aber auch andere Pflanzenfasern, wie Leinen, Hanf, Jute und Ramiefasern; regenerierte Celluloserfasern sind beispielsweise Zellwolle und Viskosekunstseide.

Carbonamidgruppenhaltige Materialien sind beispielsweise synthetische und natürliche Polyamide und Polyurethane, insbesondere in Form von Fasern, wie beispielsweise Wolle und andere Tierhaare, Seide, Leder, Polyamid-6,6, Polyamid-6, Polyamid-11 und Polyamid-4.

Aminogruppenhaltige Materialien sind ebenso beispielsweise Polyamide, wie synthetische Polyamide aus der Reihe Polyamid-6,6, Polyamid-4, Polyamid-11 und Polyamid-6, oder natürliche Proteinfasern, wie Wolle, Seide, Leder und Tierhaare, und insbesondere durch Aminogruppen modifizierte Cellulosefasern, wie durch Aminogruppen modifizierte Baumwolle.

Die Farbstoffe (1) lassen sich auf verschiedene Weise auf das Fasermaterial applizieren und auf der Faser fixieren, insbesondere in Form von wäßrigen Farbstofflösungen und -druckpasten. Solche Verfahren sind zahlreich aus der Literatur bekannt (s. bspw. die europäische Patentanmeldungs-Veröffentlichung Nr. 0 181 585). Sie eignen sich sowohl für die allgemein bekannten Ausziehverfahren in weiten Temperaturbereichen, wie bei 40 bis 90 ° C, bevorzugt 60 bis 80 ° C, als auch zum Färben nach dem Foulard-Färbeverfahren, wonach die Ware mit der wäßrigen Farbstofflösung imprägniert und der Farbstoff nach einer Alkalibehandlung oder in Gegenwart von Alkali, gegebenenfalls unter Wärmeeinwirkung, bspw. durch Dämpfen, fixiert wird. Bevorzugt werden die Farbstoffe (1) in die Ausziehverfahren eingesetzt. Hierbei erhält man auch

dann hochwertige Färbungen, wenn das Färbebad nur eine geringe Menge an einem oder mehreren Elektrolytsalzen, wie Natriumchlorid, Kaliumchlorid und Natriumsulfat, beispielsweise 20 bis 40 g/l Färbeflotte im Gegensatz zu den in der Technik allgemein verwendeten Mengen von 50 bis 80 g/l, enthält. Bevorzugt finden sie auch in den Klotz-Kaltweil-Verfahren ihre färberische Anwendung. Nach dem Fixieren werden die Färbungen oder Drucke mit kaltem und heißem Wasser, gegebenenfalls unter Zusatz eines dispergierend wirkenden und die Diffusion der nicht fixierten Anteile fördernden Mittels, gründlich gespült.

Die Farbstoffe (1) zeichnen sich durch hohe Reaktivität, gutes Fixiervermögen und ein gutes Aufbauvermögen aus. Die erhältlichen Färbungen besitzen eine hohe Egalität. Die Fixiergrade sind hoch und nicht fixierte Anteile können leicht ausgewaschen werden. Die Farbstoffe (1) eignen sich auch zum Druck, vor allem auf Baumwolle, ebenso aber auch zum Bedrucken von stickstoffhaltigen Fasern, zum Beispiel von Wolle oder Seide oder von Mischgeweben, die Wolle oder Seide enthalten.

Die mit den Farbstoffen (1) hergestellten Färbungen und Drucke besitzen, insbesondere auf Cellulosefasermaterialien, eine hohe Brillanz, eine hohe Farbstärke und eine hohe Faser-Farbstoff-Bindungsstabilität sowohl in saurem als auch in alkalischem Bereich, weiterhin eine gute Lichtechtheit und sehr gute Naßechtheitseigenschaften, wie Wasch-, Wasser-, Seewasser-, Ueberfärbe- und Schweißechtheiten, sowie eine gute Plissierechtheit, Bügelechtheit und Reibechtheit.

Die nachfolgenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter.

Die in den Beispielen formelmäßig beschriebenen Verbindungen sind in Form der freien Säure angegeben; im allgemeinen werden sie in Form ihrer Alkalimetallsalze, wie Lithium-, Natrium- oder Kaliumsalze, hergestellt und isoliert und in Form ihrer Salze zum Färben verwendet. Ebenso können die in den nachfolgenden Beispielen, insbesondere Tabellenbeispielen, in Form der freien Säure genannten Ausgangsverbindungen und Komponenten als solche oder in Form ihrer Salze, vorzugsweise Alkalimetallsalze, in die Synthese eingesetzt werden.

Die für die erfindungsgemäßen Farbstoffe angegebenen Absorptionsmaxima ($\lambda_{max}$) im sichtbaren Bereich wurden anhand ihrer Alkalimetallsalze in wäßriger Lösung ermittelt. In den Tabellenbeispielen sind die $\lambda_{max}$-Werte bei der Farbtonangabe in Klammern gesetzt; die Wellenlängenangabe bezieht sich auf nm.

Beispiel 1

58,9 Teile der Triphendioxazinverbindung der Formel (10)

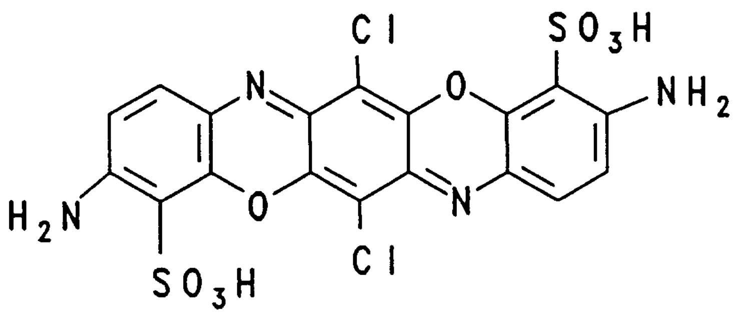

(10)

(herstellbar gemäß den Angaben der Europäischen Patentanmeldungs-Veröffentlichung Nr. 0 170 838) werden in 1500 Teilen Wasser angerührt, mittels 5 Teilen Lithiumhydroxid gelöst und mit 47,4 Teilen der Verbindung 6-{Bis-N,N-[$\beta$-($\beta$'-chlorethylsulfonyl)-ethyl]-amino}-2,4-dichlor-1,3,5-triazin bei einer Temperatur von etwa 80 °C unter Aufrechterhaltung eines pH-Wertes von 4,5 mittels einer wäßrigen Lithiumcarbonatlösung während etwa fünf Stunden unter Rühren umgesetzt.
Die erhaltene erfindungsgemäße Triphendioxazinverbindung der Formel (in Form der freien Säure geschrieben)

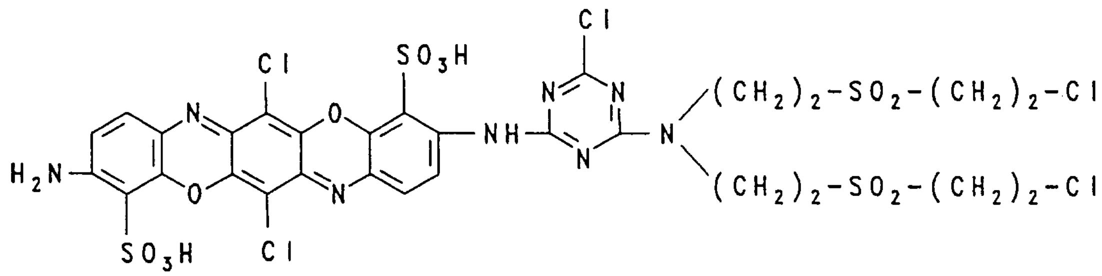

($\lambda_{max}$ = 570 nm)

wird in üblicher Weise aus der Syntheselösung durch Aussalzen mit Natriumchlorid als Natriumsalz isoliert. Sie zeigt sehr gute faserreaktive Farbstoffeigenschatten und färbt nach den in der Technik für faserreaktive Farbstoffe üblichen Färbe- und Druckverfahren die in der Beschreibung genannten Fasermaterialien, wie insbesondere Cellulosefasermaterialien, in brillanten rotstichig blauen Tönen bei hohem Fixiergrad. Nicht fixierte Reste des Farbstoffes lassen sich leicht aus der erhaltenen Färbung auswaschen. Die Färbung besitzt eine sehr hohe Egalität.

Beispiel 2

58,9 Teile des Lithiumsalzes der Triphendioxazinverbindung der Formel (10) werden in 1500 Teilen Wasser angerührt und bei einem pH-Wert zwischen 4,5 und 6 und einer Temperatur von 75 bis 85 °C mit 120 Teilen der Verbindung 6-{Bis-N,N-[$\beta$-($\beta$'-chlorethylsulfonyl)-ethyl]-amino}-2,4-dichlor-1,3,5-triazin während etwa sechs Stunden unter Rühren umgesetzt.
Man erhält die erfindungsgemäße Triphendioxazinverbindung der Formel (in Form der freien Säure geschrieben)

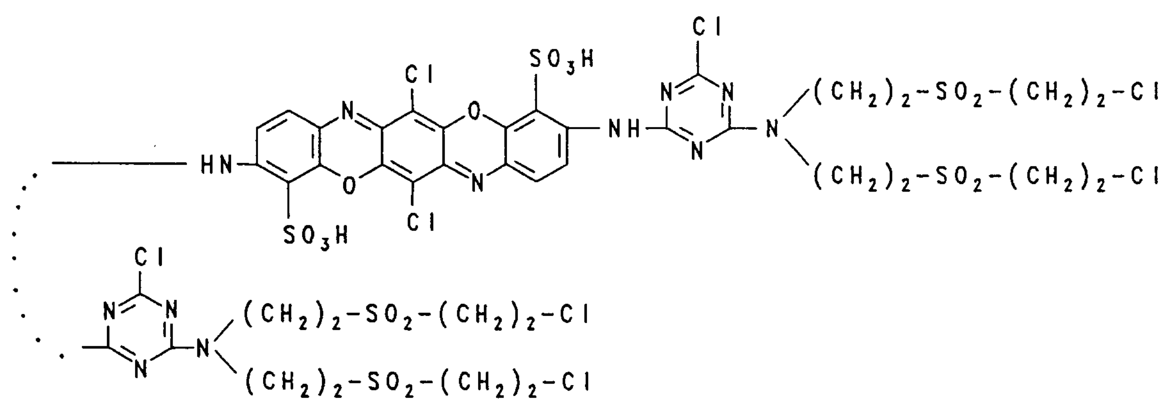

($\lambda_{max}$ = 568 nm)

in Mischung mit deren teilweise vinylisierten Form. Sie wird in üblicher Weise aus der Syntheselösung als Alkalimetallsalz isoliert, beispielsweise durch Aussalzen mittels Natriumchlorid. Die erfindungsgemäße Verbindung besitzt sehr gute faserreaktive Farbstoffeigenschaften und färbt die in der Beschreibung genannten Materialien, wie insbesondere Cellulosefasermaterialien, nach den in der Technik für faserreaktive Farbstoffe üblichen Applikations- und Fixierverfahren in brillanten rotstichig blauen Tönen mit hohem Fixiergrad. Die Färbungen weisen eine hohe Egalität auf, und nicht fixierte Reste des auf der Färbung befindlichen Farbstoffes lassen sich leicht auswaschen.

Beispiel 3

Zur Herstellung der analogen Vinylsulfonyl-Verbindung der nach Beispiel 1 erhaltenen erfindungsgemäßen Triphendioxazinverbindung versetzt man die aus Beispiel 1 erhaltene wäßrige Syntheselösung dieser erfindungsgemäßen Verbindung unter gutem Rühren bei einer Temperatur von 20 bis 25 °C mit konzentrierter wäßriger Natronlauge einem pH-Wert von 11,5 bis 12. Man rührt unter Einhaltung dieses pH-Bereiches und einer Temperatur von 20 bis 25 °C noch etwa 30 Minuten nach, stellt sodann einen pH-Wert von 6 ein und isoliert den erfindungsgemäßen Farbstoff (in Form der freien Säure geschrieben)

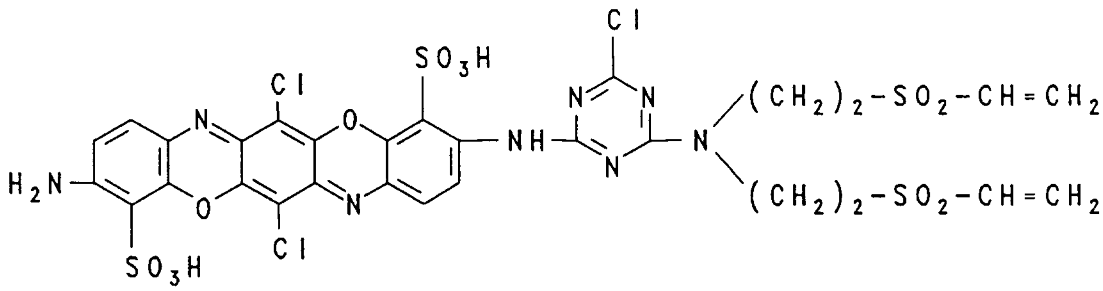

($\lambda_{max}$ = 569 nm)

aus der Syntheselösung in üblicher Weise, beispielsweise durch Aussalzen mit Natriumchlorid als Natriumsalz. Die erfindungsgemäße Vinylsulfonyl-Verbindung färbt in gleicher Weise beispielsweise Cellulosefasermaterialien in brillanten rotstichig blauen Tönen in hoher Farbstärke und mit hohem Fixiergrad.

Beispiel 4

61,7 Teile der Triphendioxazinverbindung der Formel

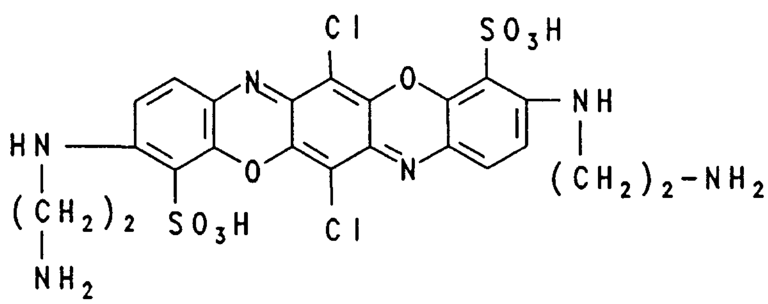

(herstellbar gemäß den Angaben der europäischen Patentanmeldungs-Veröffentlichung Nr. 0 486 429) werden in 1000 Teilen Wasser bei einem pH-Wert von 11 bis 12 mittels Lithiumhydroxid gelöst und versetzt diese langsam unter gutem Rühren mit einer auf einen pH-Wert von 6,5 bis 7 eingestellten Lösung von 98 Teilen der Verbindung 6-{Bis-N,N-[β-(β'-chlorethylsulfonyl)-ethyl]-amino}-2,4-dichlor-1,3,5-triazin in 500 Teilen Wasser, wobei man den pH-Wert des Reaktionsgemisches zunächst mit verdünnter wäßriger Essigsäure und später mit einer wäßrigen Lithiumcarbonatlösung auf einen pH-Wert zwischen 6,5 und 7 hält. Anschließend erhöht man die Reaktionstemperatur des Ansatzes auf 70 bis 80 °C und rührt den Ansatz innerhalb dieses Temperaturbereiches und einem pH-Wert zwischen 6,5 und 7 bis zur Beendigung der Umsetzung weiter.

Die synthetisierte erfindungsgemäße Triphendioxazinverbindung, die, in Form der freien Säure geschrieben, der Formel

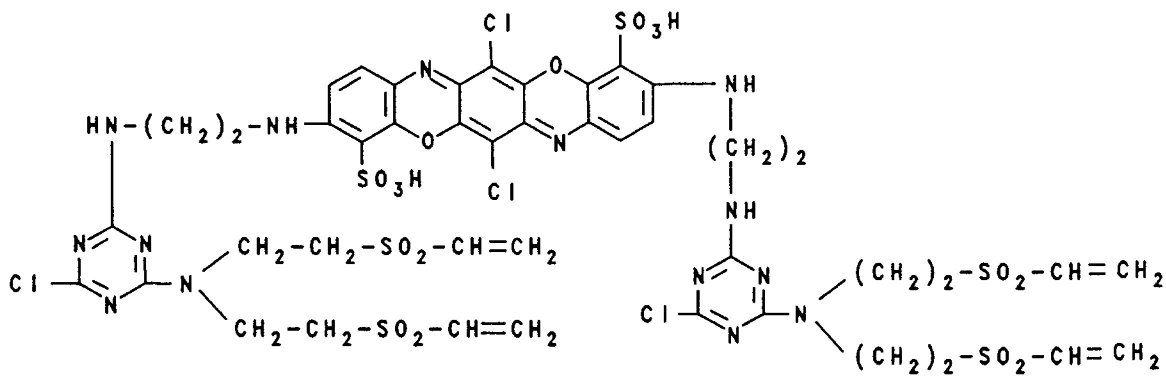

$(\lambda_{max} = 615\ nm)$

entspricht, wird in üblicher Weise, beispielsweise durch Aussalzen mit Natriumchlorid, als Alkalimetallsalz (Natriumsalz) isoliert. Sie zeigt sehr gute Farbstoffeigenschaften und liefert beispielsweise auf Cellulosefasermaterialien nach den in der Technik für faserreaktive Farbstoffe üblichen Applikations-und Fixierverfahren farbstarke Färbungen und Drucke in brillanten grünstichig blauen Tönen.

Beispiel 5

61 Teile der aus der europäischen Patentanmeldungs-Veröffentlichung Nr. 0 485 329 bekannten Triphendioxazinverbindung der Formel

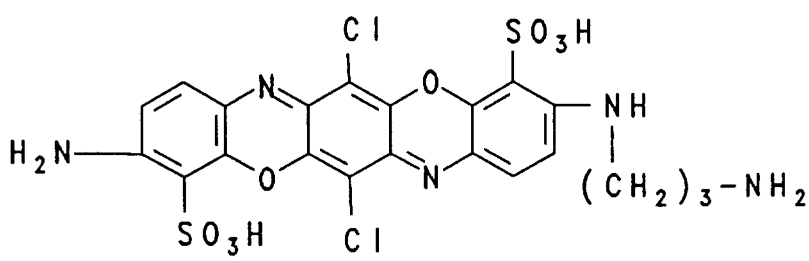

werden in 1000 Teilen Wasser bei einem pH-Wert von 10 als Lithiumsalz gelöst. Die Lösung wird langsam bei 20 bis 25 °C mit 98 Teilen der Verbindung 6-{Bis-N,N-[$\beta$-($\beta$'-chlorethylsulfonyl)-ethyl]-amino}-2,4-dichlor-1,3,5-triazin versetzt. Man rührt den Reaktionsansatz bei einem pH-Wert zwischen 8 und 9 und einer Temperatur zwischen 70 und 80 °C bis zur Beendigung der Umsetzung, kühlt sodann den Ansatz auf Raumtemperatur ab, stellt den pH-Wert auf 6 und isoliert die erfindungsgemäße Verbindung der Formel

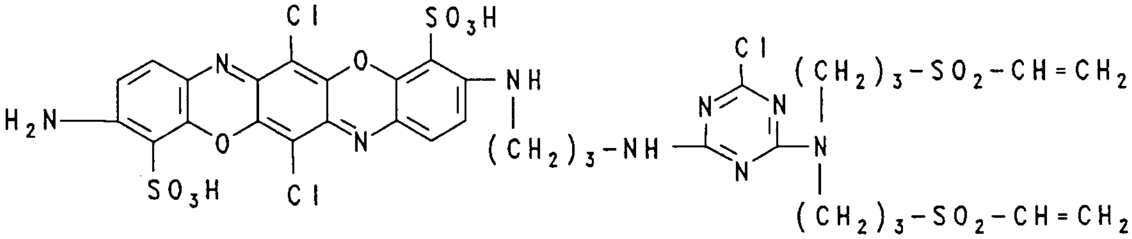

$(\lambda_{max} = 599\ nm)$

durch Aussalzen mit Natriumchlorid als Natriumsalz.

Die erfindungsgemäße Triphendioxazinverbindung zeigt sehr gute faserreaktive Farbstoffeigenschaften und färbt die in der Beschreibung genannten Materialien, wie insbesondere Baumwolle, in brillanten blauen Tönen, mit hoher Farbstärke und guten Echtheitseigenschaften.

Beispiele 6 bis 27

In den nachfolgenden Tabellenbeispielen sind weitere erfindungsgemäße Triphendioxazinverbindungen entsprechend einer allgemeinen Formel (A)

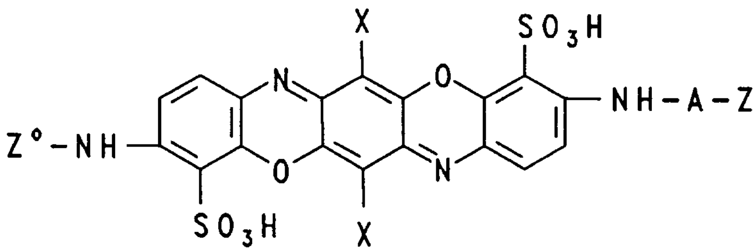

(A)

mit Hilfe ihrer Komponenten beschrieben. Sie lassen sich in erfindungsgemäßer Verfahrensweise, wie beispielsweise analog einem der obigen Beispiele, unter Einsatz der aus den Komponenten der Formel (A) ersichtlichen Ausgangsverbindungen herstellen. Sie besitzen sehr gute faserreaktive Farbstoffeigenschaften und liefern auf den in der Beschreibung genannten Materialien, wie insbesondere Cellulosefasermaterialien, nach den üblichen Anwendungsverfahren farbstarke, echte Färbungen und Druck in dem in dem jeweiligen Tabellenbeispiel angegebenen Farbton.

| | Triphendioxazinverbindung (A) | | | | |
|---|---|---|---|---|---|
| Bsp. | Rest $Z^o$ | Rest X | Rest A | Rest Z | Farbton |
| 6 | Wasserstoff | Chlor | kovalente Bindung | 4-{Bis-N,N-[γ-(ß'-chlor-ethyl-sulfonyl)-propyl]-amino}-2-chlor-1,3,5-triazin-6-yl | rotstichig blau (565) |
| 7 | Wasserstoff | Chlor | kovalente Bindung | 4-{Bis-N,N-(γ-vinyl-sulfonyl-propyl)-amino}-2-chlor-1,3,5-triazin-6-yl | rotstichig blau (566) |
| 8 | 4-{Bis-N,N-(γ-vinylsulfonyl-propyl)-amino}-2-chlor-1,3,5-triazin-6-yl | Chlor | kovalente Bindung | dito | rotstichig blau (561) |
| 9 | ß-{4-Bis-N,N-[γ'-(ß"-chlorethylsulfonyl)-propyl-amino]-2-chlor-1,3,5-triazin-6-ylamino}-ethyl | Chlor | $-CH_2-CH_2-NH-$ | 4-{Bis-N,N-[γ-(ß'-chlor-ethyl-sulfonyl)-propyl]-amino}-2-chlor-1,3,5-triazin-6-yl | grünstichig blau (616) |

| | Triphendioxazinverbindung (A) | | | | |
|---|---|---|---|---|---|
| Bsp. | Rest $Z^o$ | Rest X | Rest A | Rest Z | Farbton |
| 10 | ß-{4-Bis-N,N-[ß'-(ß"-chlor-ethylsulfonyl)-ß'-methyl-ethylamino]-2-chlor-1,3,5-triazin-6-ylamino}-isopropyl | Chlor | 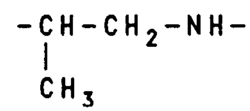 | dito | grünstichig blau (614) |
| 11 | γ-{4-Bis-N,N-[ß'-(ß"-chlorethylsulfonyl)-ethyl-amino]-2-chlor-1,3,5-triazin-6-ylamino}-propyl | Chlor | $-(CH_2)_3-NH-$ | 4-{Bis-N,N-[ß-(ß'-chlor-ethyl-sulfonyl)-ethyl]-amino}-2-chlor-1,3,5-triazin-6-yl | grünstichig blau (615) |
| 12 | 2-Sulfo-4-{4'-bis-N,N-[ß'-(ß"-chlorethylsulfonyl)-ethyl-amino]-2'-chlor-1',3',5'-triazin-6'-ylamino}-phen-1-yl | Chlor | 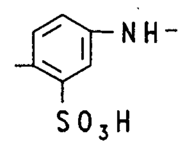 | dito | grünstichig blau (639) |

| | Triphendioxazinverbindung (A) | | | | |
|---|---|---|---|---|---|
| Bsp. | Rest $Z^o$ | Rest X | Rest A | Rest Z | Farbton |
| 13 | 3-Sulfo-4-{4'-bis-N,N-[ß'-(ß"-chlorethylsulfonyl)-ethyl-amino]-2'-chlor-1',3',5'-triazin-6'-ylamino}-phen-1-yl | Chlor | 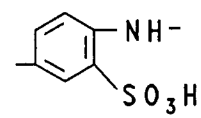 | dito | grünstichig blau (640) |
| 14 | 4-Sulfo-3-{4'-bis-N,N-[ß'-(ß"-chlorethylsulfonyl)-ethyl-amino]-2'-chlor-1',3',5'-triazin-6'-ylamino}-phen-1-yl | Chlor | 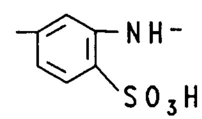 | dito | grünstichig blau (635) |
| 15 | 6-Sulfo-3-{4'-bis-N,N-[ß'-(ß"-chlorethylsulfonyl)-ethyl-amino]-2'-chlor-1',3',5'-triazin-6'-ylamino}-phen-1-yl | Chlor | 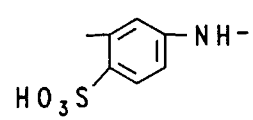 | dito | grünstichig blau (633) |

| | Triphendioxazinverbindung (A) | | | | |
|---|---|---|---|---|---|
| Bsp. | Rest $Z^o$ | Rest X | Rest A | Rest Z | Farbton |
| 16 | Wasserstoff | Wasserstoff | kovalente Bindung | dito | rotstichig blau (569) |
| 17 | Wasserstoff | Brom | kovalente Bindung | dito | blau (580) |
| 18 | Wasserstoff | Methyl | kovalente Bindung | dito | rotstichig blau |
| 19 | Wasserstoff | Ethoxycarbonyl | kovalente Bindung | dito | blau (570) |
| 20 | Wasserstoff | Phenyl | kovalente Bindung | dito | violett ((561) |
| 21 | Wasserstoff | Phenoxy | kovalente Bindung | dito | blau (571) |
| 22 | Wasserstoff | Methoxy | kovalente Bindung | dito | blau (569) |
| 23 | Wasserstoff | Chlor | $-(CH_2)_2-NH-$ | dito | blau (600) |
| 24 | Wasserstoff | Chlor | $-(CH_2)_2-NH-$ | 4-{Bis-N,N-[γ-(ß'-chlor-ethylsulfonyl)-propyl]-amino}-2-chlor-1,3,5-triazin-6-yl | blau (601) |

| | Triphendioxazinverbindung (A) | | | | |
|---|---|---|---|---|---|
| Bsp. | Rest $Z^o$ | Rest X | Rest A | Rest Z | Farbton |
| 25 | 4-{Bis-N,N-[γ-(ß'-chlorethyl-sulfonyl)-propyl]-amino}-2-chlor-1,3,5triazin-6-yl | Chlor | kovalente Bindung | dito | rotstichig blau (560) |
| 26 | Wasserstoff | Chlor | $-(CH_2)_3-NH-$ | 4-{Bis-N,N-[ß-(ß'-chlor-ethylsulfonyl)-ethyl]-amino}-2-chlor-1,3,5-triazin-6-yl | blau (598) |
| 27 | Wasserstoff | Chlor | $-(CH_2)_3-NH-$ | 4-{Bis-N,N-[γ-(ß'-chlor-ethylsulfonyl)-propyl]-amino}-2-chlor-1,3,5-triazin-6-yl | blau (600) |

## Patentansprüche

1. Triphendioxazinverbindung der allgemeinen Formel (1)

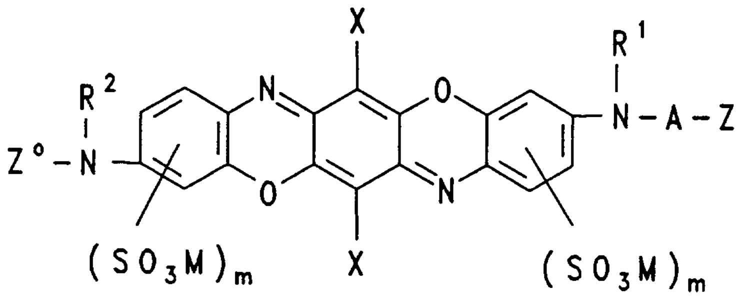

(1)

in welcher bedeuten:

M ist Wasserstoff oder ein Alkalimetall;

m ist die Zahl 1 oder 2;

X ist Wasserstoff, Halogen, gegebenenfalls substituiertes Alkyl von 1 bis 4 C-Atomen, gegebenenfalls substituiertes Aryl, gegebenenfalls substituiertes Aryloxy, Cyano, Alkoxycarbonyl von 2 bis 5 C-Atomen, Aryloxycarbonyl, Aminocarbonyl, N-Alkyl-aminocarbonyl mit einem Alkylrest von 1 bis 4 C-Atomen, N,N-Dialkylaminocarbonyl mit Alkylresten von jeweils 1 bis 4 C-Atomen, gegebenenfalls substituiertes Alkylcarbonyl von 2 bis 5 C-Atomen oder gegebenenfalls substituiertes Arylcarbonyl;

$R^1$ ist Wasserstoff, gegebenenfalls substituiertes Alkyl von 1 bis 6 C-Atomen oder gegebenenfalls substituiertes Aryl;

$R^2$ hat eine der für $R^1$ angegebene Bedeutungen;

Z ist ein Rest der allgemeinen Formel (2)

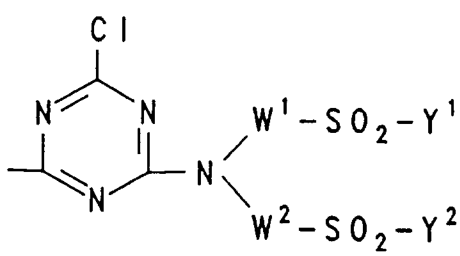

(2)

in welcher

$Y^1$ Vinyl ist oder Ethyl ist, das in $\beta$-Stellung einen Substituenten enthält, der mittels Alkali unter Bildung der Vinylgruppe eliminierbar ist, oder $\beta$-Hydroxyethyl ist,

$Y^2$ eine der Bedeutungen von $Y^1$ hat mit der Maßgabe, daß $Y^1$ und $Y^2$ nicht gleichzeitig $\beta$-Hydroxyethyl bedeuten,

$W^1$ Alkylen von 2 bis 6 C-Atomen ist und

$W^2$ eine der für $W^1$ angegebenen Bedeutungen hat;

A ist eine kovalente Bindung oder eine Gruppe der allgemeinen Formel (3a)

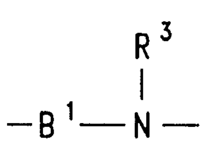

(3a)

in welcher

$R^3$ eine der für $R^1$ angegebenen Bedeutungen besitzt und

$B^1$ Alkylen von 2 bis 6 C-Atomen ist oder Alkylen von 3 bis 10 C-Atomen ist, das durch 1 oder 2 Heterogruppen, wie Gruppen der Formeln -O-, -NH-, -S-, $-SO_2-$, -CO-, -NH-CO-, $-NH-SO_2-$, $-SO_2-NH-$, -CO-NH-, -NH-CO-NH-, -NH-CO-O-, -CO-O-, -O-OC-NH-, -O-OC- oder -N(R)-mit R gleich Alkyl von 1 bis 4 C-Atomen, unterbrochen ist, oder Cycloalkylen von 5 bis 8 C-Atomen ist oder ein Rest der Formel phen , naphth , alk-phen , phen-alk, naphth-alk, alk-naphth , phen-alk-phen , alk-phen-alk, phen-D-phen , alk-D-phen , phen-D-alk, cy-alk- , alk-cy, cy-alk-cy oder alk-cy-alk, in welchen phen Phenylen bedeutet, das durch 1 oder 2 Substituenten aus der Gruppe Methyl, Ethyl, Methoxy, Sulfo und Carboxy substituiert sein kann, naphth Naphthylen bedeutet, das durch 1 oder 2 Sulfogruppen substituiert sein kann, alk Alkylen von 1 bis 6 C-Atomen bedeutet, das durch 1 oder 2 Substituenten aus der Gruppe Hydroxy, Acetyloxy, Sulfo, Carboxy oder Sulfato substituiert und/oder durch eine der obengenannten Heterogruppen unterbrochen sein kann, cy Cycloalkylen von 5 bis 8 C-Atomen ist und D eine der obengenannten Heterogruppen bedeutet, oder

die Gruppe $-N(R^1)-B^1-N(R^3)-$ stellt insgesamt einen bivalenten gesättigten heterocyclischen Rest dar;

$Z^0$ ist Wasserstoff oder ist eine Gruppe der allgemeinen Formel (4)

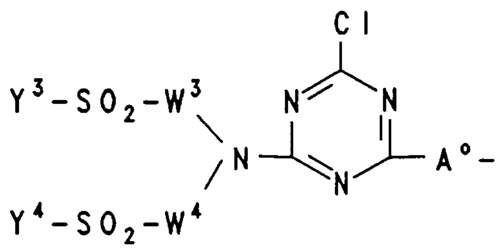

(4)

in welcher

$Y^3$ und $Y^4$ eine der oben für $Y^1$ genannten Bedeutungen haben;

$W^3$ und $W^4$ eine der oben für $W^1$ genannten Bedeutungen haben;

$A^0$ eine kovalente Bindung oder ein Rest der allgemeinen Formel (3b)

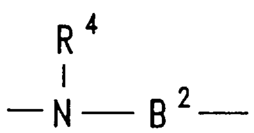

(3b)

ist, in welcher

$R^4$ eine der oben für $R^1$ angegebenen Bedeutungen hat und

$B^2$ eine der oben für $B^1$ genannten Bedeutungen besitzt, oder die Gruppe $-N(R^4)-B^2-N(R^2)-$ stellt einen bivalenten gesättigten heterocyclischen Rest dar.

2. Verbindung nach Anspruch 1, dadurch gekennzeichnet, daß m die Zahl 1 ist.

3. Verbindung nach Anspruch 1 oder 2, dadurch gekennzeichnet, daß X beide für Chlor stehen.

4. Verbindung nach mindestens einem der Ansprüche 1 bis 3, dadurch gekennzeichnet, daß $W^1$, $W^2$, $W^3$ oder $W^4$, zueinander gleich oder voneinander verschieden, jedes für Ethylen oder Propylen steht.

5. Farbstoff nach mindestens einem der Ansprüche 1 bis 4, dadurch gekennzeichnet, daß $Y^1$, $Y^2$, $Y^3$ und $Y^4$, zueinander gleich oder voneinander verschieden, jedes für $\beta$-Chlorethyl oder Vinyl steht.

6. Verbindung nach mindestens einem der Ansprüche 1 bis 5, dadurch gekennzeichnet, daß $R^1$, $R^2$, $R^3$ und $R^4$ jedes Wasserstoff bedeuten.

7. Farbstoff nach Anspruch 1 der allgemeinen Formel (1A)

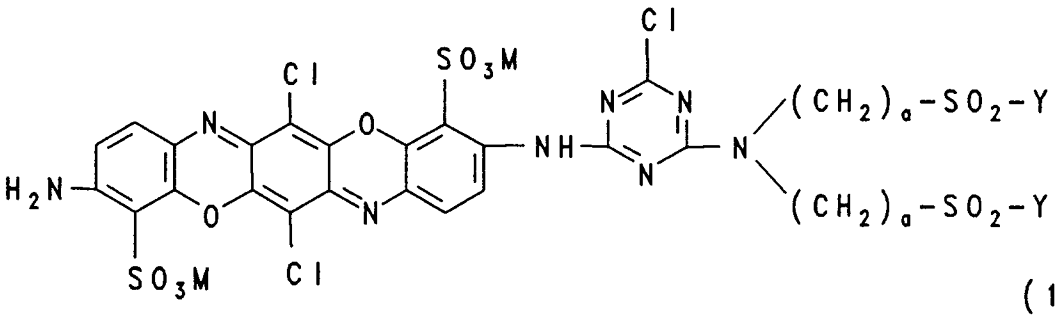

(1A)

in welcher M die in Anspruch 1 genannte Bedeutung besitzt, a für die Zahl 2 oder 3 steht und Y β-Chlorethyl oder Vinyl ist.

**8.** Verbindung nach Anspruch 1 der allgemeinen Formel (1B)

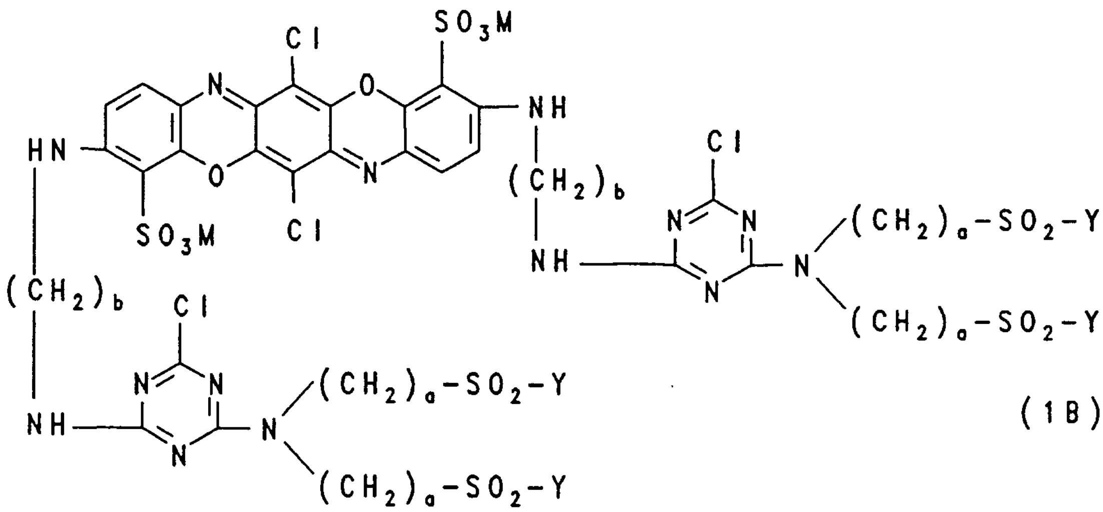

(1B)

in welcher M die in Anspruch 1 genannte Bedeutung besitzt, a für die Zahl 2 oder 3 steht, b die Zahl 2 oder 3 bedeutet und Y β-Chlorethyl oder Vinyl ist.

**9.** Verbindung nach Anspruch 1 der allgemeinen Formel (1D)

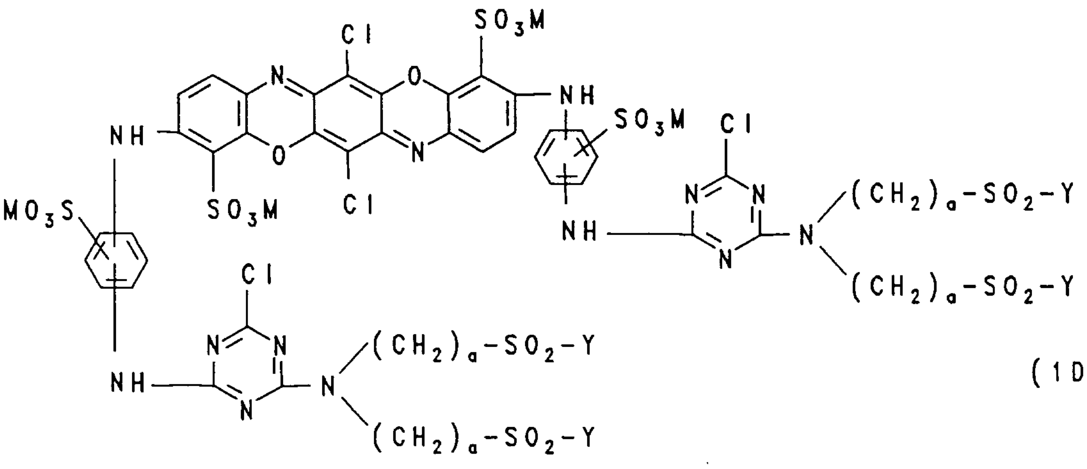

(1D)

in welcher M die in Anspruch 1 genannte Bedeutung besitzt, a für die Zahl 2 oder 3 steht und Y β-Chlorethyl oder Vinyl ist.

**10.** Verfahren zur Herstellung einer Triphendioxazinverbindung der allgemeinen Formel (1) von Anspruch 1, dadurch gekennzeichnet, daß man Cyanurchlorid in beliebiger Reihenfolge mit einer aminogruppenhaltigen Triphendioxazinverbindung der allgemeinen Formel (7)

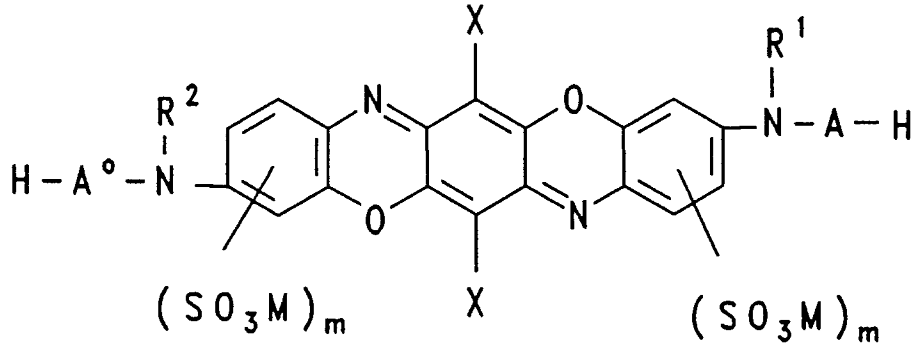

(7)

in welcher $A^0$, A, M, m, X, $R^1$ und $R^2$ die in Anspruch 1 angegebenen Bedeutungen haben, und mit einer Aminoverbindung der allgemeinen Formel (8a) und/oder (8b)

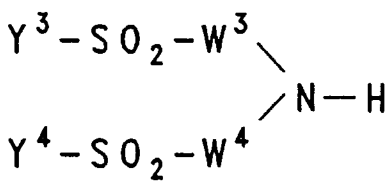

(8a)

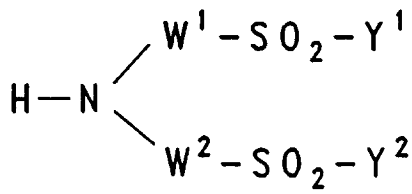

(8b)

in welcher $W^1$, $W^2$, $W^3$` $W^4$, $Y^1$, $Y^2$, $Y^3$ und $Y^4$ die in Anspruch 1 genannten Bedeutungen haben, oder mit dem Salz des Amins einer in der Technik üblichen anorganischen oder organischen Säure in der äquivalenten Menge umsetzt.

**11.** Verfahren nach Anspruch 10 zur Herstellung einer Triphendioxazinverbindung der allgemeinen Formel (1), in welcher $Z^0$ Wasserstoff und A eine kovalente Bindung ist, dadurch gekennzeichnet, daß man eine Triphendioxazinverbindung der allgemeinen Formel (7), in welcher $A^0$ und A beide eine kovalente Bindung bedeuten, mit einer Dichlortriazinylaminoverbindung der allgemeinen Formel (9)

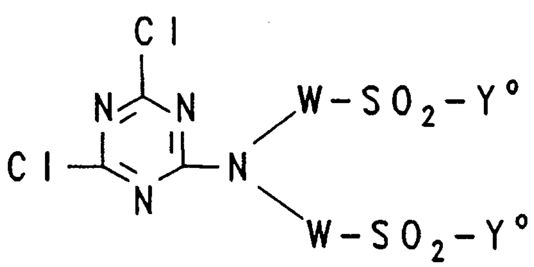

(9)

in welcher jedes W, zueinander gleich oder voneinander verschieden, und jedes $Y^0$, zueinander gleich oder voneinander verschieden, eine der in Anspruch 1 genannten Bedeutungen von $W^1$ und $W^2$ bzw. $Y^1$ und $Y^2$ haben, bei einer Temperatur zwischen 60 und 95 °C und bei einem pH-Wert von 4 bis 5 umsetzt.

**12.** Verwendung einer Triphendioxazin-Verbindung der allgemeinen Formel (1) von Anspruch 1 zum Färben von hydroxy- und/oder carbonamidgruppenhaltigem Material, insbesondere Fasermaterial.

**13.** Verfahren zum Färben von hydroxy- und/oder carbonamidgruppenhaltigem Material, vorzugsweise Fasermaterial, bei welchem man ein Farbstoff in wäßrigem Medium mit dem Material in Kontakt bringt und die Fixierung des Farbstoffes auf dem Material mittels Wärme oder mit Hilfe eines alkalisch wirkenden Mittels oder mit Hilfe beider Maßnahmen durchführt, dadurch gekennzeichnet, daß der Farbstoff eine Triphendioxazin-Verbindung der allgemeinen Formel (1) von Anspruch 1 ist.

**14.** Verfahren nach Anspruch 13, dadurch gekennzeichnet, daß das Verfahren zum Färben ein Auszieh-Färbeverfahren ist und die Färbeflotte einen Elektrolytsalzgehalt von insgesamt weniger als 50 g/l Färbeflotte enthält.

Europäisches Patentamt

# EUROPÄISCHER RECHERCHENBERICHT

Nummer der Anmeldung
EP 95 10 6233

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | DE-A-26 14 550 (CASSELLA FARBWERKE MAINKUR AG) 27.Oktober 1977 * Ansprüche 1,2,10-17,19,20,27; Beispiel 1 * | 1-6,12, 13 | C07D498/04 C09B19/02 C09B62/04 D06P3/66 |
| P,X | EP-A-0 629 667 (HOECHST AG) 21.Dezember 1994 * Ansprüche 1-15 * | 1-14 | |
| D,X | EP-A-0 074 928 (CIBA-GEIGY AG) 23.März 1983 * Seite 4, Zeile 21 - Seite 5, Zeile 5; Ansprüche 1-4 * | 1-14 | |
| D,Y | EP-A-0 070 807 (CIBA-GEIGY AG) 26.Januar 1983 * Seite 3, Zeile 1 - Seite 3, Zeile 3; Ansprüche 1-8,11,13,14 * | 1-13 | |
| Y | EP-A-0 170 838 (BAYER AG) 12.Februar 1986 * Seite 3, Zeile 1 - Seite 3, Zeile 3; Ansprüche 1-4,6 * | 1-13 | |

RECHERCHIERTE SACHGEBIETE (Int.Cl.6)

C07D
C09B
D06P

Der vorliegende Recherchenbericht wurde für alle Patentansprüche erstellt

| Recherchenort | Abschlußdatum der Recherche | Prüfer |
|---|---|---|
| MÜNCHEN | 18.September 1995 | Herz, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument